# EUROPEAN PATENT APPLICATION

(11) **EP 4 775 257 A1**
(43) Date of publication of application: **15.07.2026**
(21) Application number: 26150901.2
(22) Date of filing: 08.01.2026
(51) Int. Cl.: A61N 1/05, A61N 1/372, A61N 1/375, A61N 1/362

(54) **BIOSTIMULATOR HAVING EXTENDIBLE ELECTRODE**

(30) Priority: 10.01.2025 US 202563744070 P; 07.01.2026 US 202619442850
(71) Applicant: Pacesetter, Inc., Sylmar, CA 91342 (US)
(72) Inventor: TEAGUE, Bryan, Sylmar, 91342 (US); CHANTASIRIVISAL, Steve, Sylmar, 91342 (US); ALLEMAN, Wesley, Sylmar, 91432 (US); MANALO, Megan, Sylmar, 91342 (US); WELCH, Mark, Sylmar, 91342 (US); VICTORINE, Keith, Sylmar, 91342 (US)
(74) Representative: Barker Brettell Sweden AB

(57) **Abstract**

An implantable biostimulator (104, 200, 302) has a housing (106, 212, 402, 502, 602), one or more fixation elements (210, 304, 418, 510, 610) at a distal end of the housing (106, 212, 402, 502, 602), and one or more electrode probes (208, 306, 406, 506). The housing (106, 212, 402, 502, 602)includes an electronics compartment containing pacing circuitry. The one or more electrode probes (208, 306, 406, 506)are coupled to the pacing circuitry. The one or more electrode probes (208, 306, 406, 506)are extendable from the distal end of the housing (106, 212, 402, 502, 602). The one or more electrode probes (208, 306, 406, 506) have depth control to a controlled depth of extension.

## Description

### TECHNICAL FIELD

The present disclosure relates to biostimulators. More specifically, the present disclosure relates to leadless biostimulators having tissue anchors and electrode probes.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Cardiac pacing by currently available or conventional pacemakers is usually performed by a pulse generator implanted subcutaneously or sub-muscularly in or near a patient's pectoral region. The generator usually connects to a proximal end of one or more implanted leads, the distal end of which contains one or more electrodes for positioning adjacent to the inside or outside wall of a cardiac chamber. Although more than one hundred thousand conventional cardiac pacing systems are implanted annually, various well-known difficulties exist, and there is an ongoing need for improvement in the art.

### SUMMARY

Various embodiments, variations, and examples are herein described for an implantable biostimulator, and components thereof which may include a housing, may include fixation element(s), and may include electrode probe(s). The implantable biostimulator may be a leadless biostimulator, such as a leadless pacemaker.

In one embodiment, an implantable biostimulator includes a housing, one or more fixation elements, and one or more electrode probes. The housing includes an electronics compartment. The electronics compartment contains pacing circuitry. The fixation elements are at a distal end of the housing. The electrode probe or probes are coupled to the pacing circuitry. The electrode probe or probes are extendable from the distal end of the housing. The electrode probe or probes have depth control to a controlled depth of extension.

The above summary does not include an exhaustive list of all aspects of the present invention. It is contemplated that the invention includes all devices, and systems that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 depicts a leadless implantable medical device, biostimulator or pacemaker (i.e., leadless pacemaker) implanted in the interventricular septal wall of a heart.
FIG. 2 is a profile view of a leadless biostimulator or pacemaker in an embodiment featuring a fixation helix, and multiple electrode probes that are deployable via a movable annular ring.
FIG. 3 is a profile view of the distal end of a leadless biostimulator or pacemaker in an embodiment featuring passive tines and a single electrode probe.
FIGS. 4A-4B depict the distal end of a leadless biostimulator or pacemaker in an embodiment featuring an electrode probe with ball features.

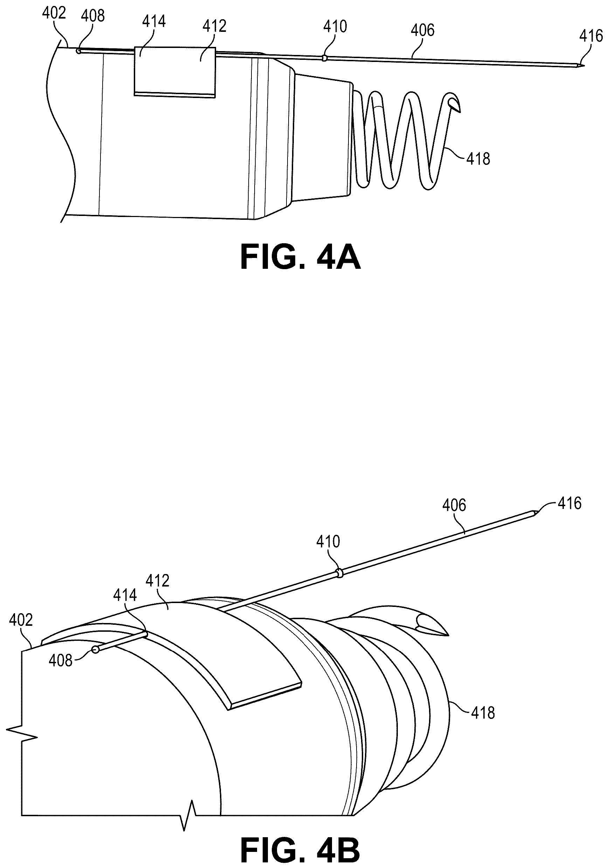

FIG. 4A is a profile view of the distal end of the embodiment, featuring the electrode probe with ball features.
FIG. 4B is a perspective view of the distal end of the embodiment, featuring the electrode probe with ball features.

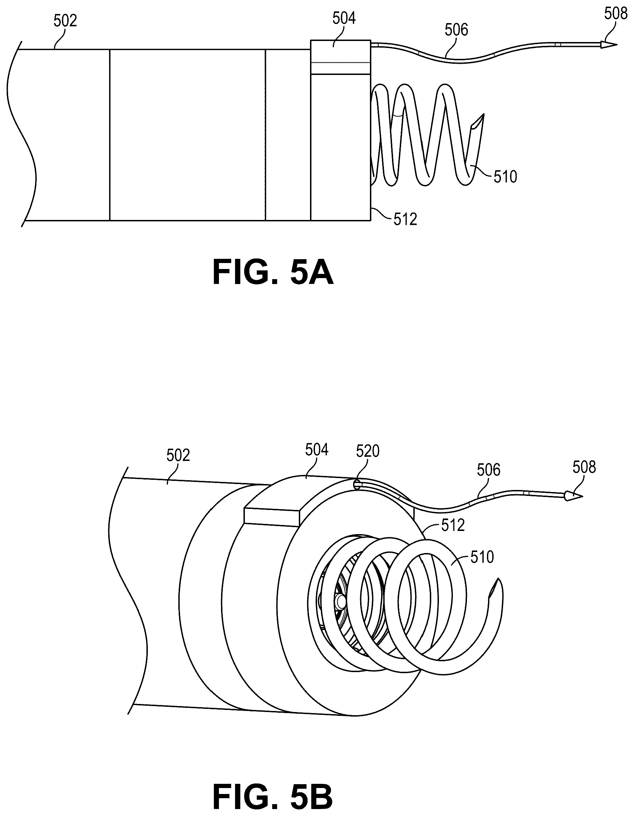

FIGS. 5A-D depict the distal end of a leadless biostimulator or pacemaker, in embodiments featuring a flexible electrode.
FIG. 5A is a profile view of the distal end of the biostimulator in an embodiment, featuring the flexible electrode deployed from a radiopaque marker of the housing.
FIG. 5B is a perspective view of the distal end of the biostimulator in an embodiment, featuring the flexible electrode deployed from a radiopaque marker of the housing.

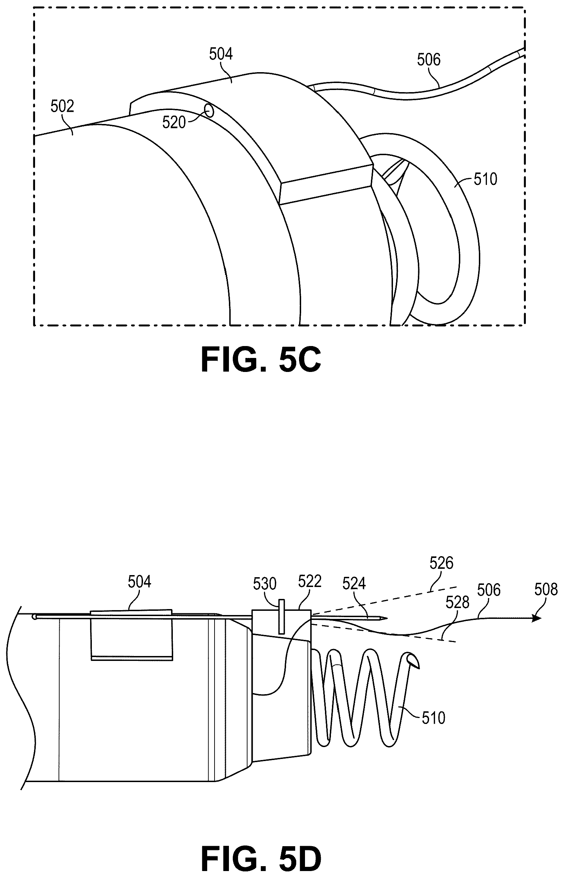

FIG. 5C is a further perspective view of the distal end of the biostimulator in an embodiment, featuring the flexible electrode deployed through a hypotube deployment hole.
FIG. 5D is a further profile view of the distal end of the biostimulator in an embodiment, featuring the flexible electrode and a range of deployment angles for further consideration in variations.

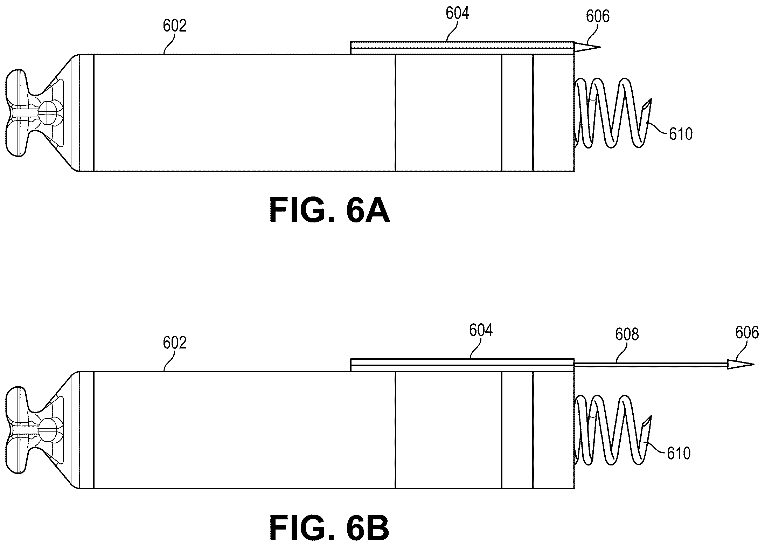

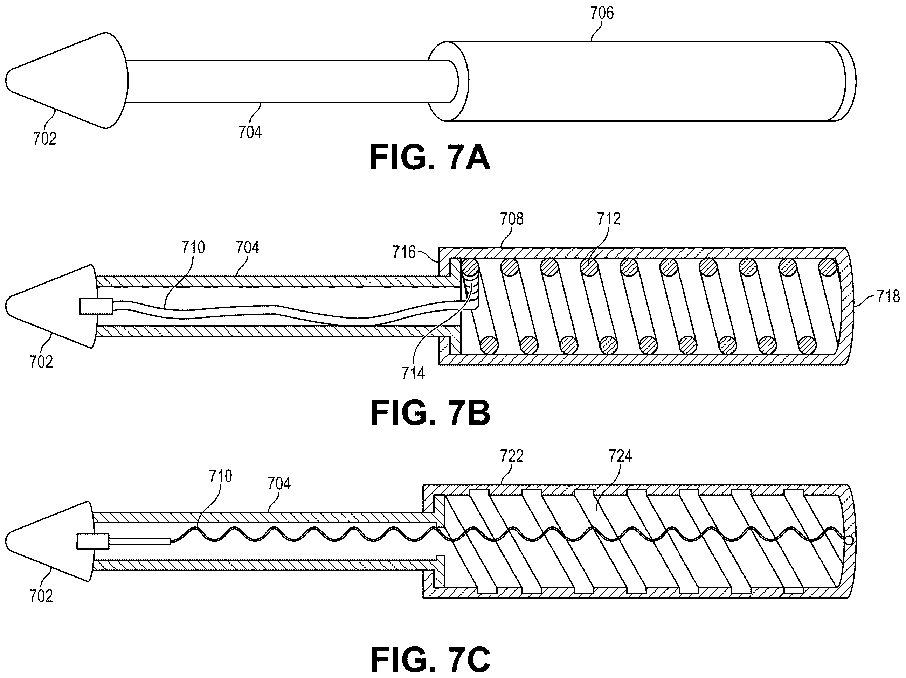

FIGS. 6A-6C and 7A-7C are profile views of a leadless biostimulator or pacemaker, in embodiments featuring a telescoping electrode.
FIG. 6A depicts the telescoping electrode in a retracted state.
FIG. 6B depicts the telescoping electrode in an extended state.

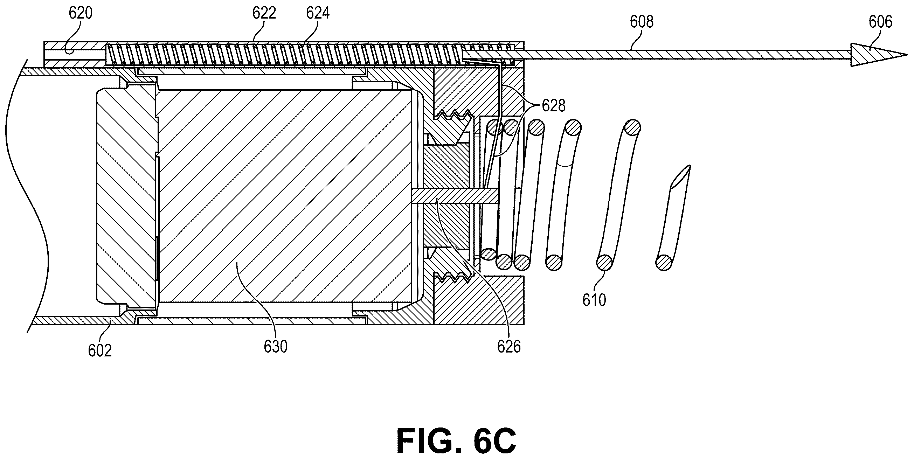

FIG. 6C is a cutaway view of the distal end of a leadless biostimulator or pacemaker, in embodiments or variations of an embodiment featuring a telescoping electrode with a telescoping mechanism that may be considered spring-loaded, threaded, or both.
FIG. 7A depicts the telescoping electrode, showing the electrode probe tip, an electrode shaft, and a tube, in an embodiment.
FIG. 7B is a cutaway view of the telescoping electrode, in an embodiment featuring a spring or spring-loading.
FIG. 7C is a cutaway view of the telescoping electrode, in an embodiment featuring threading or a threaded tube.

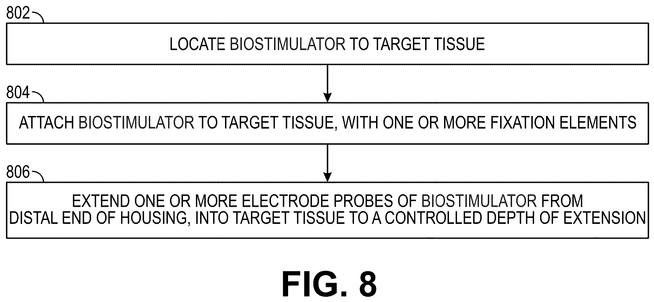

FIG. 8 is a flowchart of a method of treatment using a biostimulator.

### DETAILED DESCRIPTION

Described herein are embodiments of a biostimulator, which may be a leadless implantable medical device, a leadless cardiac pacemaker, or leadless biostimulator for implantation to stimulate biological tissue, such as nerves or muscle, for example heart tissue, brain tissue, nerve bundles, or muscle tissue. Transport systems, for use in implantation and retrieval of the biostimulator, including a biostimulator delivery system and a biostimulator retrieval system, are described. The biostimulator transport system can be used to deliver or retrieve a biostimulator, e.g., a cardiac pacemaker, from a heart of a patient. The biostimulator may, however, be used in other applications, such as deep brain stimulation. Thus, reference to the biostimulator as being a cardiac pacemaker is not limiting.

Embodiments of a biostimulator feature fixation element(s) and electrode probe(s). Some embodiments feature an active fixation helix, for the fixation element. Some embodiments feature passive tines, for the fixation elements. Some embodiments feature one electrode probe, some embodiments feature multiple electrode probes. In some embodiments, the electrode probe(s) are extendable and have depth control to a controlled depth of extension. In some embodiments, the electrode probe(s) are extendable and retractable. Various mechanisms for extension and/or retraction are described. Various mechanisms for depth control of electrode probe extension are described. The terms "rigid" and "flexible" are relative to a given embodiment and capabilities thereof. For example, a "rigid" hypotube is more rigid and less flexible than a "flexible" electrode.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative portion of an element, a relative position, or relative direction. For example, "distal" may indicate a first direction along a central axis of a biostimulator or a biostimulator transport system. Similarly, "proximal" may indicate a second direction opposite to the first direction. Such terms are provided to establish relative frames of reference, however, and are not intended to limit the use or orientation of a biostimulator or a biostimulator transport system to a specific configuration described in the various embodiments below.

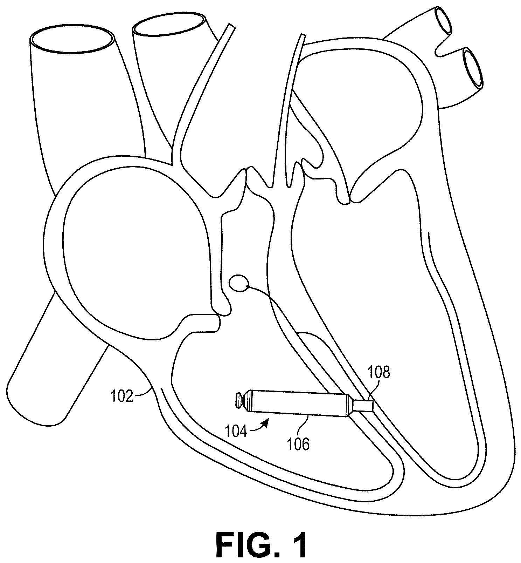

FIG. 1 depicts a leadless implantable medical device, biostimulator 104 or pacemaker (i.e., leadless pacemaker) implanted in the interventricular septal wall of a heart 102. Although this implantation location is specific to the drawing and related surgical procedure, FIG. 1 serves to illustrate more general and widely applicable advantages of the various embodiments further described below.

Physiological pacing at left bundle branch area pacing (LBBAP) provides many clinical benefits. Leadless pacing technology continues to grow in treating pacemaker indication. Implanting a leadless pacemaker or biostimulator 104 in the interventricular septal wall, as shown in FIG. 1, provides many challenges due to the weight and geometric constraint of a cylindrical housing 106. While certain structures of the biostimulator 104 are provided in as a block illustration, such as a pacing tip 108, it will be appreciated that the structures are representative of any of the embodiments of the biostimulator 104 described below.

One major obstacle in this space, is the ability to place the pacing tip 108 at the appropriate septal depth for a large patient population. The present disclosure discusses novel fixation elements or features to aid in implanting the pacing tip depth for a pacemaker system (e.g., for a leadless pacemaker or a biostimulator). For example, a leadless biostimulator can have a right-hand wound coil that acts as the fixation helix. When viewed along an axis of the device from the proximal end, the device is rotated clockwise to affix the coil or fixation helix into the heart wall. For the purposes of this disclosure, a similar right-hand wound coil is shown as a fixation helix. However, other forms of fixation are possible in these embodiments, such as passive, flexible tines, and other methods. All mentions of clockwise/counterclockwise in this document refer to this orientational convention unless otherwise noted, but could work with all directions reversed in the same manner, in various embodiments.

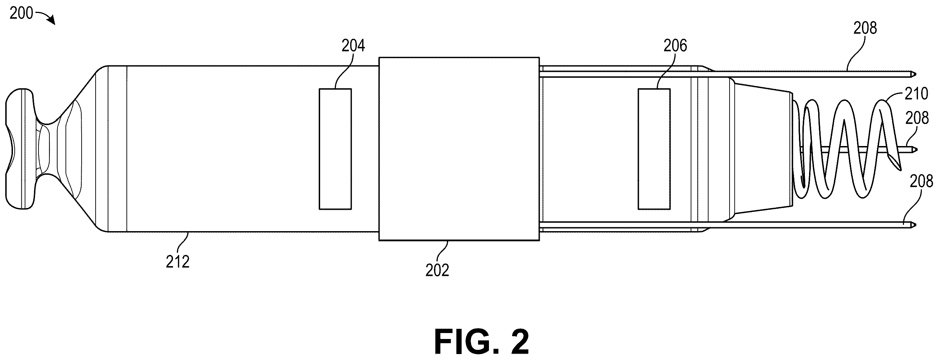

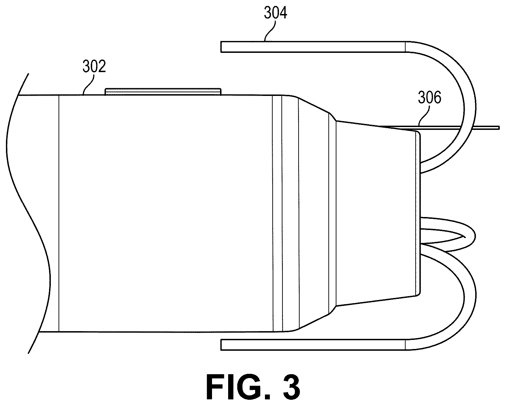

FIG. 2 is a profile view of a leadless biostimulator 200 or pacemaker in an embodiment featuring a fixation helix 210, and multiple electrode probes 208 that are deployable via a movable annular ring 202. These embodiments encompass an annular ring 202 installed around the outside surface of the biostimulator 200. The ring 202 may be moved forward (distal) and backward (proximal) along the axial length of the biostimulator 200 by use of a corresponding component located in the device catheter (not shown but readily understood as part of a biostimulator delivery system) after the device has been fixated on the septum. The device may be fixated on the septum through rotation with an active fixation helix or retention of passive tines 304 as shown in FIG. 3, or other method of fixation. There are multiple probe tips, e.g., distal ends of electrode probes 208, spaced circumferentially around the annular ring 202. For example, there are three electrode probes 208 and corresponding probe tips as shown, though more or less may be used in various embodiments. Each electrode probe 208 extends from a distal end of the annular ring 202, with a proximal end of the electrode probe 208 connected to or at the distal end of the annular ring 202, and a distal end of the electrode probe 208 featuring a respective probe tip. Moving the ring 202 forward (distal direction) advances or extends the electrode probe(s) 208, moving the ring 202 backward (proximal direction) retracts the electrode probe(s) 208. Embodiments with multiple probe tips may allow multiple probe deployments in the septal tissue in order to increase the likelihood of LBB capture.

Electrically, in some embodiments, this biostimulator 200 with multiple probe tips has one feedthrough pin per probe tip and the biostimulator 200 is programed with selective probe activation to allow it to select which electrode probe 208 to activate based on electrograms (EGMs). Selective probe activation may be accomplished with switching electronics, in various embodiments. In some embodiments, the electrode probes 208 are coated with parylene or other insulator or insulative material, except for probe tips, to function as bipolar electrodes, enabling the visualization for localized EGMs. These signals can display LBB morphology or fascicular signals, allowing for a more precise targeted area. Additionally, each of the electrode probes 208 around the annular ring 202 may be the same length (as shown) or different lengths to allow for multiple locations/depths (and corresponding depth control) when trying to locate the LBB. Finally, the biostimulator 200 has additional "stopping pads" 204, 206 mounted to the external cylindrical face of the housing 212 to act as hard stops on the depth control of the electrode probes 208. The retracted pad or stopping pad 204 (retracted) is located such that the electrode probes 208 are not deployed in septal tissue when the biostimulator 200 is being fixated to the septum, and the extended pad or stopping pad 206 (extended) is located such that the electrode probes 208 are fully deployed in septal tissue after the biostimulator 200 has been fixed in the septum.

FIG. 3 is a profile view of the distal end of a leadless biostimulator 302 or pacemaker in an embodiment featuring passive tines 304 and a single electrode probe 306. Passive tines 304 may be used in place of a fixation helix, for presently described and further embodiments of the biostimulators or pacemakers. In some embodiments, passive tines 304 are pointed or have a sharp tip, similar to the tip of the fixation helix shown in some embodiments. Various mechanisms for implantation using passive tines 304 may be known or readily devised for use therewith. Passive tines 304, or fixation tines, may be advantageous in some applications where it is desired to not rotate the biostimulator 302, e.g., when attempting a second or repeat placement of an electrode, or when one of multiple electrodes may be selected for activation. In contrast, in applications where it is desired to rotate the biostimulator, a fixation helix may be advantageous, e.g., when rotating the biostimulator allows a different site for a second or repeat placement of an electrode.

Passive tines 304 are preshaped and self-expandable, in some embodiments. When constrained, the tines 304 are straightened. But when advanced and unconstrained, the tines 304 may curl into the shape seen in FIG. 3, e.g., when anchoring in tissue.

FIGS. 4A-4B depict the distal end of a leadless biostimulator or pacemaker in an embodiment featuring an electrode probe 406 with ball features 408, 410, also referred to as balls herein. One embodiment of a leadless biostimulator or pacemaker encompasses a semi-flexible probe 406 having several ball features 408, 410 along a probe length. One ball 408 is fixated at the proximal end of the electrode probe 406 and a second ball 410 is fixated at a specified length distal to the proximal end of the electrode probe 406 (e.g., 10 mm). Appropriate dimensions for length of electrode probe 406 and placement of ball features 408, 410 relate to depth control for the electrode probe 416. Between these two fixed balls 408, 410, a side-mounted pad or flange 412 on a body of the biostimulator, e.g., on the housing 402, constrains a portion of the electrode probe 406 with a through hole 414, also referred to as a guide hole 414, that has a smaller diameter than the two balls 408, 410. Various further shapes may be used in further embodiments.

FIG. 4A is a profile view of the distal end of the embodiment, featuring the electrode probe 406 with ball features 408, 410. The guide hole 414 is indicated in the dashed lines through the pad or flange 412, which is mounted to or integral with the housing 402.

FIG. 4B is a perspective view of the distal end of the embodiment, featuring the electrode probe 406 with ball features 408, 410. One ball 408, can be located at a proximal end of the electrode probe 406 to constrain maximum extension of the electrode probe 406. Also, one ball 410, located along a midsection of the electrode probe 406 can constrain maximum retraction of the electrode probe 406. The guide hole 414 is indicated at an opening along an edge of the pad or flange 412 in this view.

The biostimulator in this example is fixated to the septum (e.g., through rotation with an active fixation helix 418 or retention of passive tines, or other method of fixation), and then the electrode probe 416 is deployed in the distal axial direction through a distinct action by use of a mating component in the implant catheter (e.g., a dilator or other grasping/retention/release and/or manipulating mechanism within the catheter itself, not shown but readily understood as part of an operation and mechanism of a biostimulator transport system). The two balls 408, 410 on either side of the pad or flange 412 can prevent the electrode probe 406 from becoming separated from the biostimulator, and can prevent over-deployment, e.g., moving through the entire septal wall and penetrating the blood pool of the left ventricle (LV). An added benefit of this design and related embodiments, when incorporated with an active fixation helix 418, is the ability to "reposition" multiple attempts without removing the fixation from the septal wall. Once the biostimulator is secured and in place, the electrode probe 416 may be deployed into the septum to the appropriate depth (i.e., with depth control), and then retracted if the implant location does not offer appropriate conductive system pacing. The biostimulator may then be rotated multiple times (e.g., up to one additional revolution in total) and the electrode probe 416 may be re-deployed at any point during that additional revolution for additional attempts without incurring the tissue damage of a full relocation of the device along a different point on the septal wall.

FIGS. 5A-D depict the distal end of a leadless biostimulator or pacemaker, in embodiments featuring a flexible electrode 506 as an embodiment of an electrode probe. A radiopaque marker 504 is also shown, and it should be appreciated that these two features may be used in combination or one without the other in various further embodiments. For example, a radiopaque marker 504 is visible in x-rays or x-ray imaging, and may be used during surgery to guide implantation and deployment of this or other types of electrodes. A flexible electrode 506 may have various advantages with or without a radiopaque marker 504. The combination of the two may be advantageous in guiding implantation of a flexible electrode 506 through visibility of a radiopaque marker 504 during surgery.

FIG. 5A is a profile view of the distal end of the biostimulator in an embodiment, featuring the flexible electrode 506 deployed from a radiopaque marker 504 of the housing 502. The flexible electrode 506 may be segmented or smooth and uniform in composition, in various embodiments. In some embodiments, the flexible electrode 506 has a cable structure or is a wire with an ETFE (ethylene tetrafluoroethylene), PTFE (polytetrafluoroethylene) or other fluoropolymer or Teflon-based coating or insulator, with the electrode tip 508 exposed, uncoated, or uninsulated. Shape, dimensions and even curvature of the radiopaque marker 504 may be an aid to orientation determination by the surgeon or other technician, to guide in accuracy of placement of the electrode tip 508 and flexible electrode 506 during surgery. A fixation helix 510 is visible projecting from a helix mount 512 of the housing 502. Location (e.g., at distal end of the housing) and orientation of the radiopaque marker 504 may aid orientation determination of the fixation helix 510 during fixation to tissue, in surgery.

FIG. 5B is a perspective view of the distal end of the biostimulator in an embodiment, featuring the flexible electrode 506 deployed from a radiopaque marker 504 of the housing 502. Further details of a portion of the fixation helix 510 within the helix mount 512 are visible at this view. Projected contours of the radiopaque marker 504, for x-rays or x-ray imaging, are readily envisioned in relationship to deployment of the flexible electrode 506 and electrode tip 508.

FIG. 5C is a further perspective view of the distal end of the biostimulator in an embodiment, featuring the flexible electrode 506 deployed through a hypotube deployment hole 520, see also FIG. 5B. The hypotube deployment hole 520 can act as a guide hole, e.g., in the manner of guide hole 414. For example, not shown but readily envisioned, a hypotube could be deployed through the hypotube deployment hole 520, and used as a rigid sheath of or for the flexible electrode 506, to deploy the electrode tip 508. Consider the flexible electrode 506 sheathed by a hypotube, with the electrode tip 508 projecting from a distal end of the hypotube. This assembly is deployed in a forward or distal direction through the hypotube deployment hole 520, guiding the electrode tip 508 into a target tissue to a controlled depth. Then, the hypotube is retracted back to or back into the hypotube deployment hole 520, leaving the flexible electrode 506 with electrode tip 508 deployed in the target tissue.

Embodiments shown in FIGS. 5A-D encompass a radiopaque marker 504 with a small through hole, also referred to as a hypotube deployment hole 520, mounted to the side of the fixation helix mount 512. In some embodiments, the feedthrough flange and fixation helix mount 512 are the same diameter as the battery of the biostimulator, and the mounted radiopaque marker 504 increases the overall outer diameter on only a small portion of a distal circumference of the biostimulator. For the flexible electrode 506, a small, flexible insulated cable or wire is strung from the electrical feedthrough, through the fixation helix mount 512 and radiopaque marker 504, and runs distally out of the through hole, e.g., hypotube deployment hole 520 in the radiopaque marker 504. The tip 508 of the flexible electrode 506 is pointed to reduce resistance when inserting into the septal wall. Once in place, a hypotube (or similar structure built into the delivery catheter) is inserted into the through hole 520 in the radiopaque marker 504, and is used as a rigid guide to push the flexible electrode 506 into the septal tissue to the appropriate depth to capture the LBB.

FIG. 5D is a further profile view of the distal end of the biostimulator in an embodiment, featuring the flexible electrode 506 and a range of deployment angles (shown in dashed lines 526, 528) for further consideration in variations. It may be desirable to control or constrain a deployment angle for the flexible electrode 506 sheathed by a hypotube, so that the tip of the flexible electrode 506 is properly placed in a desired location in a target tissue. A deployment angle may be visualized as a cone of projection. For such functionality, some embodiments employ a director 530, which could be a guide plate with an aperture or guide block with a through hole, manually positionable prior to surgery or perhaps mechanically operable during surgery. For example, there could be a cylindrical hole, or a conical hole, and a straight versus an angled guide, which may also be termed a steering guide or deflection guide. There may be a mechanical selector in the handle of a catheter or other biostimulator delivery system, which operates the director 530 in the biostimulator. The director 530 constrains motion and direction, more specifically deployment angle, for a sheathed flexible electrode 524, e.g., rigid hypotube sheathing a flexible electrode. For example, the director 530 may determine whether the hypotube with flexible electrode 506 is deployed straight ahead, or deflected to an angle, e.g., in a range. The director 530 is depicted enclosed by a housing 522, which may protect this mechanism and/or prevent fouling of tissue during surgery. The director 530 may be considered a steering, guiding or track mechanism, i.e., a directing mechanism, for deployment of a hypotube and flexible electrode 506. Related to other embodiments, the radiopaque marker 504 may be used advantageously to determine orientation and guide deployment of the electrode 506, with or without a director 530 as the case may be.

With reference to FIGS. 5A-D, the flexible electrode 506 may be sufficiently flexible to support heartbeats or other tissue movement while the electrode 506 is anchored in tissue. The hypotube may act as a stiffening member, for example being composed of a thin-walled metallic or stainless steel tube like a hypodermic needle or hypodermic tube.

FIGS. 6A-6C and 7A-7C are profile views of a leadless biostimulator or pacemaker, in embodiments featuring a telescoping electrode. These embodiments and variations thereof encompass an offset spring-loaded, threaded, or other extension mechanism telescoping arm. Embodiments also include the option of active or passive fixation. The offset component may be akin to a cylinder piston which includes an outer piston bore housing and inner piston, which is electrically connected to the feedthrough pin. The inner bore may be either spring-loaded or threaded, e.g., may have a spring or a screw thread channel, though other configurations are possible. Once fixated, a stylet from the catheter (not shown, but readily envisioned as operation and part of a mechanism of a biostimulator delivery system) enters the proximal side of the outer piston housing and pushes or turns (e.g., like a screw) the inner piston to extend and penetrate the septal wall until it extends to the appropriate depth into the septum to get LBB capture and conductive system pacing. Once the appropriate depth is reached, the catheter-mounted stylet is removed and the extended inner piston is held in place via a set screw, or spring-force, or other method or mechanism to keep it at the appropriate position.

FIG. 6A depicts the telescoping electrode in a retracted state. The electrode tip 606 protrudes from a tube 604, which encloses the electrode shaft 608 (see FIG. 6B), ready for deployment of the electrode. Here, the tube 604 is shown mounted to the housing 602 of the biostimulator, but could be integral with or otherwise arranged in further embodiments.

FIG. 6B depicts the telescoping electrode in an extended state. That is, the electrode shaft 608 with electrode tip 606 at a distal end, protrudes from the tube 604. More specifically, a portion of the electrode shaft 608 may remain for retention in the tube 604 while a further portion of the electrode shaft 608 projects from the tube 604, in the extended state, for implantation. A fixation helix 610 is shown in this embodiment, and passive tines could be used in further embodiments.

FIG. 6C is a cutaway view of the distal end of a leadless biostimulator or pacemaker, in embodiments or variations of an embodiment featuring a telescoping electrode with a telescoping mechanism that may be considered spring-loaded, threaded, or both. An access hole 620 in a proximal end of the tube 622, such as tube 604 in FIGs. 6A and 6B, allows engagement of a catheter or other biostimulator delivery system or portion thereof, which may engage the tube 622 itself, e.g., to rotate the tube 622, or may engage the springs 624, e.g., to push or rotate the spring 624, or may engage a proximal end, e.g., a piston end of an electrode shaft 608, e.g., to control or release that end of the electrode shaft 608 for spring-loaded or spring-driven motion. Thus, the electrode probe, including electrode shaft 608 and electrode tip 606, may be deployed by spring action, screw thread action, or both. Variations limited to spring action, or limited to screw thread action, are readily understood and devised.

A wire 628 or other electrical connection can connect the electrode (e.g., electrode shaft 608 and electrode tip 606) to a feedthrough pin 626, which connects to electronics for the cathode, e.g., in an electronics bay 630 within the housing 602 of the biostimulator.

FIG. 7A depicts the telescoping electrode, showing the electrode probe tip 702, an electrode shaft 704, and a tube 706, in an embodiment. It is understood other shapes may be devised for these components. In some embodiments, the electrode probe tip 702 is electrically conductive, and the electrode shaft 704 is internally electrically conductive in connection with the electrode probe tip 702, but externally insulative. This may be accomplished with a wire as shown and described below.

FIG. 7B is a cutaway view of the telescoping electrode, in an embodiment featuring a spring or spring-loading. A spring 712 is constrained and compressible within a tube 708, such a tube 706 in FIG. 7A, with a distal end of the spring 712 contacting a base or flange of the electrode shaft 704. A collar 716 of the tube 708 constrains the base or flange of the electrode shaft 704, so that the electrode shaft 704 is not removable from the tube 708, and is spring-loaded for deployment. An end cap 718 of the proximal end of the tube 708 constrains the proximal end of the spring 712. For electrical connection, a wire 710 connects the electrode probe tip 702, to a spring tab 714. For example, the spring tab 714 can include a conductive shim extending radially from the wire 710 to the spring 712. Further electrical connections are readily devised. It is understood the dimensions and constraints of these components are used for depth control in deployment of the electrode. The electrode is extendable by telescoping action and spring-loading. Some embodiments may be retractable, for example by pulling on the wire 710, or pulling on the electrode shaft 704, etc.

FIG. 7C is a cutaway view of the telescoping electrode, in an embodiment featuring threading or a threaded tube 722, such as tube 706 in FIG. 7A. Threading or threads 724 of the tube 722 engage or are engaged by a proximal end of the electrode shaft 704, for example one or more pins or a base threaded portion of the electrode shaft 704, a spiral guided piston, a threaded feature, etc. Rotating the tube 722, with the electrode shaft 704 constrained to not rotate, or rotating the electrode shaft 704 with the tube 722 constrained to not rotate, causes screw thread driven extension or retraction of the electrode, which is also described as telescoping action. That is, rotation of one relative to the other causes the components to screw in or screw out, thus telescoping the assembly. In some embodiments, the wire 710 may be pre-twisted so it is fully untwisted when the electrode is fully extended.

FIG. 8 depicts a method of treatment using a biostimulator 104. The method may be practiced using embodiments of biostimulators 104 described herein, and variations. The method may be practiced using a biostimulator delivery system with a biostimulator 104.

In an action 802, the biostimulator 104 is located to a target tissue. For example, a leadless pacemaker is located to a target heart tissue. This may be accomplished using a biostimulator transport system, in surgery. As described above, the biostimulator transport system can be a biostimulator delivery system or a biostimulator retrieval system. The biostimulator transport system may be a catheter-based system having a handle, an elongated catheter extending distally from the handle, and a distal portion. For example, the distal portion may include a docking end or a snare configured to engage or capture a proximal end of the biostimulator 104. In an embodiment, a biostimulator system includes the biostimulator 104 mounted on the distal portion of the biostimulator transport system. The biostimulator transport system may therefore be navigated through an anatomy to control deliver to, or retrieval from, a target tissue.

In an action 804, the biostimulator 104 is attached to the target tissue, with one or more fixation elements. For example, the fixation element may be a fixation helix. The fixation element may be a passive tine or passive tines. This may be accomplished using a biostimulator delivery system, with a biostimulator 104.

In an action 806, one or more electrode probes of the biostimulator 104 are extended from the distal end of the housing of the biostimulator 104, into the target tissue to a controlled depth of extension. This may be accomplished with various embodiments described herein, that have one electrode probe, or more electrode probes, and various mechanisms for extending electrode probe(s) to a controlled depth of extension. This may be accomplished using a biostimulator delivery system, with a biostimulator.

An aspect relates to a biostimulator 104, 200, 302 comprising a housing 106, 212, 402, 502, 602 including an electronics compartment containing pacing circuitry. The biostimulator 104, 200, 302 also comprises one or more fixation elements 210, 304, 418, 510, 610 at a distal end of the housing 106, 212, 402, 502, 602. The biostimulator 104, 200, 302 further comprises one or more electrode probes 208, 306, 406, 506, coupled to the pacing circuitry, extendable from the distal end of the housing 106, 212, 402, 502, 602 to a controlled depth of extension.

In an embodiment, the one or more fixation elements 210, 304, 418, 510, 610 comprises one or more of a fixation helix 210, 418, 510, 610 or a plurality of tines 304.

In an embodiment, the one or more electrode probes 208 are based at an annular ring 202.

In an embodiment, the annular ring 202 is movably mounted to an exterior of the housing 212. In this embodiment, the housing 212 further comprises at least one stopping pad 204, 206 to provide the depth control by stop-limiting movement of the annular ring 202.

In an embodiment, the at least one stopping pad 204, 206 comprises a first stopping pad 206 that limits movement of the annular ring 202 to an electrode probe extended position.

In an embodiment, the at least one stopping pad 204, 206 comprises a second stopping pad 204 that limits movement of the annular ring 202 to an electrode probe retracted position.

In an embodiment, the one or more electrode probes 406 are each movably constrained by a guide hole 414 of a pad 412 or a flange 412 of the housing 418.

In an embodiment, each of the one or more electrode probes 406 has at least one ball feature 408, 410, to provide the depth control by stop-limiting movement of the electrode probe 406 relative to the guide hole 414.

In an embodiment, the at least one ball feature 408, 410, of each of the one or more electrode probes 406, comprises a first ball feature 410 that limits movement of the electrode probe 406 to an electrode probe retracted position, and a second ball feature 408 that limits movement of the electrode probe 406 to an electrode probe extended position.

In an embodiment, the one or more electrode probes each have a telescoping electrode operable to have a retracted state and an extended state.

In an embodiment, the housing 602 has a tube 604, 622, 706, 708 with a spring 624, 712 therein and with a proximal end of the telescoping electrode engaging the spring 624, 712 for extension of the telescoping electrode.

In an embodiment, a distal end of the tube 622, 708 is arranged to constrain the spring 624, 712 and provide the controlled depth of extension of the telescoping electrode.

In an embodiment, the housing has a threaded tube 722.

In an embodiment, the threaded tube 722 includes a proximal end engaging a thread 724 of the threaded tube 722. In this embodiment, end limits of threading of the threaded tube 722 provide the controlled depth of extension of the telescoping electrode.

Another aspect relates to a biostimulator system comprising a biostimulator transport system and the biostimulator 104, 200, 302 according to the invention mounted on a distal portion of the biostimulator transport system.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (104, 200, 302), comprising:
a housing (106, 212, 402, 502, 602) including an electronics compartment containing pacing circuitry;
one or more fixation elements (210, 304, 418, 510, 610) at a distal end of the housing (106, 212, 402, 502, 602); and
one or more electrode probes (208, 306, 406, 506), coupled to the pacing circuitry, extendable from the distal end of the housing (106, 212, 402, 502, 602) to a controlled depth of extension.

2. The biostimulator of claim 1, wherein the one or more fixation elements (210, 304, 418, 510, 610) comprises one or more of a fixation helix (210, 418, 510, 610) or a plurality of tines (304).

3. The biostimulator of claim 1 or 2, wherein the one or more electrode probes (208) are based at an annular ring (202).

4. The biostimulator of claim 3, wherein the annular ring (202) is movably mounted to an exterior of the housing (212), and further comprising at least one stopping pad (204, 206) to provide the depth control by stop-limiting movement of the annular ring (202).

5. The biostimulator of claim 4, wherein the at least one stopping pad (204, 206) comprises a first stopping pad (206) that limits movement of the annular ring (202) to an electrode probe extended position.

6. The biostimulator of claim 5, wherein the at least one stopping pad (204, 206) comprises a second stopping pad (204) that limits movement of the annular ring (202) to an electrode probe retracted position.

7. The biostimulator of claim 1 or 2, wherein the one or more electrode probes (406) are each movably constrained by a guide hole (414) of a pad (412) or a flange (412) of the housing (418).

8. The biostimulator of claim 7, wherein each of the one or more electrode probes (406) has at least one ball feature (408, 410), to provide the depth control by stop-limiting movement of the electrode probe (406) relative to the guide hole (414).

9. The biostimulator of claim 8, wherein the at least one ball feature (408, 410), of each of the one or more electrode probes (406), comprises a first ball feature (410) that limits movement of the electrode probe (406) to an electrode probe retracted position, and a second ball feature (408) that limits movement of the electrode probe (406) to an electrode probe extended position.

10. The biostimulator of claim 1 or 2, wherein the one or more electrode probes each have a telescoping electrode operable to have a retracted state and an extended state.

11. The biostimulator of claim 10, wherein the housing (602) has a tube (604, 622, 706, 708) with a spring (624, 712) therein and with a proximal end of the telescoping electrode engaging the spring (624, 712) for extension of the telescoping electrode.

12. The biostimulator of claim 11, wherein a distal end of the tube (622, 708) is arranged to constrain the spring (624, 712) and provide the controlled depth of extension of the telescoping electrode.

13. The biostimulator of claim 1 or 2, wherein the housing has a threaded tube (722).

14. The biostimulator of claim 13, wherein the threaded tube (722) includes a proximal end engaging a thread (724) of the threaded tube (722), and wherein end limits of threading of the threaded tube (722) provide the controlled depth of extension of the telescoping electrode.

15. A biostimulator system, comprising:
a biostimulator transport system; and
the biostimulator (104, 200, 302) of any of claims 1-14 mounted on a distal portion of the biostimulator transport system.
